# EUROPEAN PATENT APPLICATION

(11) **EP 4 410 264 A1**
(43) Date of publication of application: **07.08.2024**
(21) Application number: 23305122.6
(22) Date of filing: 31.01.2023
(51) Int. Cl.: A61K 8/04, A61K 8/73, A61Q 19/00

(54) **ACRYLATE-FREE COSMETIC GEL COMPOSITION AND USE THEREOF FOR SKIN CARE**

(71) Applicant: Pierre Fabre Dermo-Cosmétique, 81500 Lavaur (FR)
(72) Inventor: CHAVES, Fernanda, 81106 CASTRES (FR); AMADO, Juliana, 81106 CASTRES (FR); PEREIRA, Nayara, 81106 CASTRES (FR)
(74) Representative: Ipside

(57) **Abstract**

The invention relates to a cosmetic composition intended for the care of the skin of a subject, which is in the form of an aqueous gel having a dynamic viscosity at 25°C higher than or equal to 2000 mPa.s. This composition is devoid of acrylate and comprises, in percentage by weight relative to the total weight of the composition, at least 80 % water and 0.5 to 2 % *Caesalpinia spinosa* gum.

## Description

The invention lies in the field of topical cosmetic compositions in gel form, in particular intended for skin care.

More particularly, the invention relates to an acrylate-free cosmetic composition in gel form, and the cosmetic use thereof for the cosmetic treatment of the skin. The invention also relates to a cosmetic method for treating the skin of a subject, using such a composition.

Polymers are multifunctional materials widely applied in cosmetic formulations for different purposes, including thickening, emulsifying, stabilizing, and enhancing texture properties. One of most well-known polymers used for these aims are the synthetic ones belonging to the family of polyacrylates; and the carbomers are the most used example of this family. Carbomers can be defined as high molecular weight, crosslinked polyacrylic acid polymers. They may differ by their chemical crosslinking to help address regulatory demands, increase product robustness, and improve product handling, especially to ease polymer dispersion. They are very versatile and high-performing materials. The carboxyl groups provided by the acrylic acid backbone of the polymer are responsible for many of the polymer benefits. Even at low concentrations, these materials can create safe, stable aqueous structures with a pleasant sensorial profile. Due to these properties, they have been extensively used in the field of cosmetics to develop gel-type formulations.

However, damage of the aquatic ecosystem caused by microplastics has prompted the need to find viable eco-friendly alternatives to polyacrylates, in particular in the field of cosmetics. One of the main challenges in this context is to ensure that these alternatives make it possible to produce cosmetic compositions having the same advantageous properties as the cosmetic compositions based on polyacrylates.

The present invention aims at proposing a cosmetic composition in the form of an aqueous gel which, despite being acrylate-free, has a high viscosity, as well as a similar rheological behavior to that of compositions based on polyacrylates, so that it is easy to manipulate, which is efficient for skin care, which is stable and smooth, and which has a sensory profile as close as possible to that of compositions based on polyacrylates. Another objective of this invention is that these properties are obtained using natural ingredients, respectful of the environment and non-irritating to the skin.

It is now proposed by the inventors a cosmetic composition in the form of an aqueous gel which meets these objectives.

By composition in the form of an aqueous gel, it is herein meant, in a way that is conventional in itself, a monophasic composition, characterized by an aqueous continuous phase thickened by a hydrophilic gelling agent, as opposed to a composition in the form a cream, which is multiphasic, comprising a hydrophilic phase and a lipophilic phase, and consists more particularly of an oil-in-water emulsion.

Therefore, a first object of the invention is a cosmetic composition in the form of an aqueous gel, which is suitable for topical application, which is devoid of acrylate, which has a dynamic viscosity at 25°C higher than or equal to 2000 mPa.s, preferably higher than or equal to 3000 mPa.s, and which comprises, in percentage by weight relative to the total weight of the composition, at least 80 % water, and 0.5 to 2 % *Caesalpinia spinosa* gum, as a gelling agent. Viscosity is the measurement of flow resistance. The cosmetic composition of the invention advantageously has a high enough viscosity to allow proper handling of this product by the consumer, to ease its application in the skin and to ensure its stability and the suspension of active ingredients therein.

By devoid of acrylate, it is meant that the composition is devoid of any ingredient containing at least one acrylic group or one acrylate group, including polyacrylic and polyacrylate polymers or copolymers.

The dynamic viscosity of the composition of the invention can be modulated by varying the concentration of *Caesalpinia spinosa* gum contained therein, and, when needed, of additional thickening and/or gelling agent(s) optionally also contained in the composition.

The dynamic viscosity of the composition can for example be measured with a Brookfield viscometer, at 25°C and preferably at a speed of rotation of between 10 and 100 rpm.

The composition of the invention is preferably a viscoelastic composition of the type of non-Newtonian fluids, having a combination of elastic and viscous behaviors, so that it is pleasant to use and/or handle, as it does not have a completely solid (doesn't flow at all) nor liquid (flows without any resistance) behavior.

In embodiments of the invention, the composition of the invention is preferably a viscoelastic composition of the type of non-Newtonian fluids, having a more elastic than viscous behavior.

In other embodiments of the invention, the composition of the invention is preferably a viscoelastic composition of the type of non-Newtonian fluids, having a more viscous than elastic behavior.

In other particular embodiments of the invention, the composition of the invention is preferably a viscoelastic composition of the type of non-Newtonian fluids, having equally viscous and elastic behaviors.

*Caesalpinia spinosa* gum, also known as Tara gum, is a natural thickening and gelling agent having a galactomannan chemical structure, obtained by grinding the endosperm of seeds of the *Caesalpinia spinosa* tree, which is native of South America. This gum has the advantages of a renewable, vegetal sourcing, and of being obtainable by an entirely mechanical and physical treatment, implementing no solvent. When incorporated into a topical cosmetic composition, in addition to the fact that it is well tolerated by the skin, and does not cause cutaneous irritation or sensibilization, it advantageously constitutes an active skin care ingredient, and it contributes in particular to combat skin dehydration and to preserve the hydration barrier of the skin.

The cosmetic composition of the invention, comprising *Caesalpinia spinosa* gum in the concentration range of 0.5 to 2 %, has a sensorial profile very close to that of cosmetic gel compositions comprising acrylate-based ingredients, and, in particular, a non-sticky touch.

The cosmetic composition according to the invention may further have one or more of the characteristics described below, implemented individually or in each of their technically operating combinations.

In particular embodiments of the invention, the composition is devoid of fatty ingredients, or it comprises less than 2 %, preferably less than 1 % and more preferably less than 0.5 %, by weight of fatty ingredients, relative to the total weight of the composition.

By fatty ingredient, it is herein meant, in a conventional way, any compound containing a long aliphatic chain of carbon atoms, typically of more than 14 carbon atoms, such as synthetic or vegetable oils, waxes, butter, lanolin, petrolatum, fatty acids, fatty alcohols, etc.

In particular, the composition of the invention is preferably devoid of perfumes / oily fragrances or contains less than 0.5% and preferably less than 0.1 % by weight of such ingredients, relative to the total weight of the composition.

More generally, the composition of the invention is preferably substantially devoid of oily phase.

The composition of the invention is also preferably devoid of emulsifiers. It is in particular preferably devoid of glucoside-based emulsifiers, such as the products marketed under the name Montanov^{®} L, for example devoid of mixtures of arachidyl alcohol and behenyl alcohol and arachidyl glucoside, or mixtures of cetearyl alcohol and coco glucoside.

In particular embodiments of the invention, the composition of the invention is also devoid of any vegetable gum other than the *Caesalpinia spinosa* gum. In particular, the composition is devoid of *Acacia Senegal* gum and/or devoid of xanthan gum.

In alternative particular embodiments of the invention, the composition comprises xanthan gum, preferably as the sole additional gelling agent of the composition.

It has been discovered by the inventors that, surprisingly, although gel compositions based on xanthan gum as the sole gelling agent have too low a viscosity and an unsatisfactory sensory profile when applied topically to the skin, the addition of a small amount of xanthan gum to *Caesalpinia spinosa* gum advantageously increases the viscosity of the composition compared to *Caesalpinia spinosa* gum alone, at an equal total amount of gelling agent(s), this without negatively impacting the sensorial profile of the composition.

In addition, the composition is then advantageously a viscoelastic composition of the type of non-Newtonian fluids, exhibiting a behavior which is more elastic than viscous, which all the more increases its ease and pleasantness of use.

Therefore, in preferred embodiments of the invention, when the composition comprises xanthan gum, the weight ratio *Caesalpinia spinosa* gum / xanthan gum is between 75/25 and 95/5, preferably between 75/25 and 90/10, more preferably between 75/25 and 85/15, or between 80/20 and 95/5. This ratio is for example 80/20 or 90/10 or 95/5.

In particular embodiments of the invention, the composition comprises 0.01 to 1 %, preferably 0.01 to 0.50 %, more preferably 0.05 to 0.30 %, for example 0.05 to 0.20, or even 0.10 to 0.20 %, by weight, relative to the total weight of the composition, of xanthan gum. As an example, it comprises 0.15 % by weight of xanthan gum. Preferably, it then comprises 0.6 % by weight of *Caesalpinia spinosa* gum.

More generally, the composition of the invention preferably comprises 0.55 to 1 %, more preferably 0.6 to 0.8 %, by weight, relative to the total weight of the composition, of a mixture of *Caesalpinia spinosa* gum and xanthan gum.

In preferred embodiments of the invention, the composition comprises one or several additional cosmetically active ingredient(s), in particular active for skin care. By cosmetically active ingredient for skin care, it is meant an ingredient having a beneficial effect on the skin when it is applied thereto. Cosmetically active ingredients for skin care may for example improve the skin's hydration and moisture levels, nourish and/or smooth and/or soften the skin, protect it against oxidative stress, etc.

In particular embodiments of the invention, the composition comprises one or several humectant(s), which advantageously improve the surface moisturization of the skin and help preserve the skin surface hydrolipid film.

These humectants, also denoted as moisturizing agents, may be selected from glycerin, propylene glycol, butylene glycol, pentylene glycol, isopropyl pyroglutamate, sodium L-pyroglutamate, heptyl undecylenate, panthenol, alpha hydroxy acids, beta hydroxy acids, jojoba oil propanediol esters, and any of the mixtures thereof. Glycerin is particularly preferred, as it has the advantages of being formed naturally during the natural hydrolysis of the triglycerides of the surface hydrolipid film and of the intercorneocyte lipids of the *Stratum corneum,* and of having a strong water retention capacity at the skin surface, and therefore a strong capacity for maintaining surface moisturization.

Therefore, in particular embodiments of the invention, the composition comprises, as humectant(s), at least glycerin.

The total amount of humectants in the composition is preferably of between 3 to 20 % by weight, relative to the total weight of the composition.

The composition of the invention preferably comprises at least two humectants, in particular at least a mixture of glycerin and pentylene glycol. In such preferred embodiments, each humectant is preferably present in the composition in an amount of between 1.5 and 6 % by weight, relative to the total weight of the composition.

The composition of the invention can also contain, as topically active ingredient(s), one or several emollients, for softening and smoothing the skin. By way of examples, such emollients may be selected from C12-C15 alkyl benzoate, C12-C15 alkyl lactate, coco-caprylate, coconut alkanes, dicaprylyl carbonate, diisopropyl sebacate, isopropyl myristate, squalene, undecane, and any of the mixtures thereof.

The total amount of such emollients in the composition of the invention is then preferably less than 2 %, more preferably less than 1 % and even more preferably less than 0.5 %, by weight, relative to the total weight of the composition

In particular embodiments of the invention, the composition comprises, as a cosmetically active ingredient, hyaluronic acid or a cosmetically acceptable salt thereof.

By cosmetically acceptable, it is herein meant that the component, here the salt counterion, is generally safe, non-toxic and neither biologically nor otherwise undesirable, and that it is acceptable for use in cosmetic compositions, in particular intended for topical application to human keratinous tissue, including the skin.

Any conventional cosmetically acceptable salt of hyaluronic acid can be used according to the invention. Examples include sodium, potassium, calcium, etc., salts.

Preferably, the composition comprises sodium hyaluronate, which, owing to its capacity of deep penetration into the skin, has a strong hydrating effect, as well as the effect of reducing wrinkles and promoting firmness of the skin.

In preferred embodiments of the invention, hyaluronic acid or the cosmetically acceptable salt thereof has a molecular weight of between 700 and 1500 kDa. The amount of hyaluronic acid or a cosmetically acceptable salt thereof contained in the composition of the invention is preferably of between 0.1 and 1 %, more preferably between 0.01 and 0.5 %, and for example between 0.1 and 0.3 %, by weight, relative to the total weight of the composition. In particular, in such low concentration ranges, it has been observed by the inventors that, surprisingly, the viscosity of the composition is particularly high. This is especially the case when the molecular weight of the hyaluronic acid or the cosmetically acceptable salt thereof is between 700 and 1500 kDa, as more particularly recommended by the invention.

The composition of the invention can additionally or alternatively contain other cosmetically active ingredients, in particular ingredients active for skin care, by the topical route, such as for example vitamin E, ascorbyl glucoside and/or tocopheryl acetate, for their antioxidant properties, ultraviolet-ray blockers, vitamins, etc., or any mixture of such compounds.

The composition of the invention can also comprise any cosmetically acceptable excipient and/or additive that is conventional in itself for a topical cosmetic gel composition, such as preservatives, pH modifiers, additional gelling agents, thickeners, etc.

Preferentially, none of the ingredients of the composition of the invention is of the type associated with a risk of intolerance, or of the type which modifies the biology of the skin.

The composition of the invention is preferably devoid of cationic surfactants, which are known for their skin-irritating effect.

The composition of the invention is more preferably devoid of cationic surfactants and amphoteric surfactants.

More generally, the composition is preferably devoid of surfactant, either anionic, cationic, non-ionic or amphoteric.

The composition is for example preferably devoid of sorbitan olivate, cetearyl olivate, sorbitan palmitate, hydrogenated lecithin, as well as of sodium cocoyl apple amino acids. It is, for example, also preferably devoid of polysorbate 80.

In preferred embodiments of the invention, the composition is also devoid of agents capable of irritating the skin or of generating allergic reactions, such as quaternary ammoniums, chelating agents such as ethylenediaminetetraacetic acid (EDTA), ethanol, phenols, amidines, isothiazolone derivatives, parabens, mineral oils, silicones, lanolin and its derivatives, petrolatum, polyethylene glycols, sulphates, such as sodium lauryl sulphate, hydroquinone, glycolic acid, artificial dyes, C12-C16 alcohols, sodium gluconate, cross-polymers or copolymers.

It is also preferably devoid of polyhydroxy acids.

The composition of the invention is preferably a leave-on composition, i.e., a composition which does not require to be removed by rinsing, in particular with water, as opposed to aqueous solutions comprising surfactants or any other cleansing ingredient. The composition of the invention is intended to be left on the skin after its application thereon.

The composition of the invention can be prepared by any method known to the person skilled in the art. It is preferably prepared by introducing its ingredients, consecutively or simultaneously, in water, preferably under stirring. These operations can be carried out at ambient temperature or at a higher temperature, under heating.

Another aspect of the invention is the use of a composition according to the invention for the cosmetic, non-therapeutic, treatment of the skin of a subject, in particular a human.

This cosmetic treatment is in particular a care of the skin, intended for example for improving the skin's hydration and moisture levels, nourishing, softening and/or smoothing the skin, combating oxidative stress and/or skin ageing, reducing the wrinkles, etc.

To this end, the composition according to the invention can be topically applied to a surface of the skin of the subject, for example the skin of the face, of the neck, of the hands, and/or the rest of the body.

Another object of the invention is a cosmetic, non-therapeutic, method for treating the skin of a subject in need thereof, in particular for caring for the skin of said subject, for example for improving the skin's hydration and moisture levels, nourishing, softening and/or smoothing the skin, combating oxidative stress and/or skin ageing, reducing the wrinkles, etc.

This method comprises topically applying a composition according to the invention, in an amount effective for skin care, to a surface of said skin, for example the surface or part of the surface of the face, the neck, the hands, and/or the rest of the body.

Said surface of the skin is preferably freshly cleansed.

The subject is preferably a human.

The method of the invention is preferably devoid of rinsing step after the step of topically applying the composition to the surface of the skin.

The composition of the invention can be applied to the skin once or twice a day, preferably in the morning and/or before bedtime.

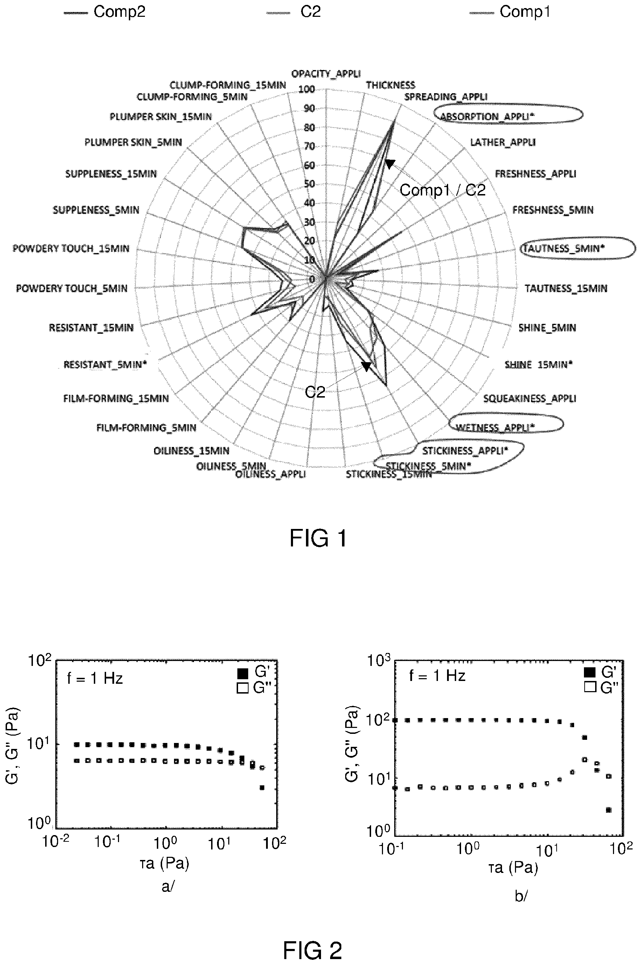

The features and advantages of the invention will emerge more clearly in the light of the following examples of implementation, provided for illustrative purposes only and in no way limitative of the invention, with the support of figures 1 and 2, in which:
- figure 1 represents the sensory profiles, as monadically assessed by 15 panelists, of respectively a composition according to the invention C2 and two comparative compositions Comp1 and Comp2, after application of these compositions on the skin;
- figure 2 represents the storage modulus G' and the loss modulus G", as a function of the stress amplitude τa, measured in a stress amplitude sweep test, for a/ a composition conform to the invention (C5) and b/ a comparative composition based on acrylate cross-polymer (Comp1).

### Example 1 - Compositions

Compositions C1 to C5 according to the invention are described in Table 1.

**Table 1 - Compositions according to the invention**

| Ingredient (INCI name) | C1 (%w/w) | C2 (%w/w) | C3 (%w/w) | C4 (%w/w) | C5 (%w/w) |
|---|---|---|---|---|---|
| Glycerine | 1.5-6 | 1.5-6 | 1.5-6 | 1.5-6 | 1.5-6 |
| Pentylene glycol | 1.5-6 | 1.5-6 | 1.5-6 | 1.5-6 | 1.5-6 |
| *Caesalpinia spinosa* gum | 1.0 | 1.0 | 1.0 | 0.6 | 0.675 |
| Xanthan gum | / | / | / | 0.15 | 0.075 |
| Water | 87-95 | 87-95 | 87-95 | 87-95 | 87-95 |
| Benzoic acid | 0.1-0.5 | 0.1-0.5 | 0.1-0.5 | 0.1-0.5 | 0.1-0.5 |
| Citric acid | 0.01-0.1 | 0.01-0.1 | / | 0.01-0.1 | 0.01-0.1 |
| Trisodium ethylenediamine disuccinate | / | / | 0.1-0.5 | / | / |
| Sodium hydroxide | / | /- | 0.1-0.5 | / | / |
| Hexanediol / caprylyl glycol | / | / | 0.5-2 | / | / |
| Sodium hyaluronate | / | 0.2 | 0.2 | / | / |
| Ascorbyl glucoside | / | / | 0.05-0.3 | / | / |
| Tocopheryl acetate | / | / | 0.05-0.3 | / | / |
| *Lens esculenta* (Lentil) Seed Extract | | / | 0.5-2 | | |

Comparative compositions Comp1 and Comp2 not in accordance with the invention are described in Table 2.

**Table 2 - Comparative compositions**

| Ingredient (INCI name) | Comp1 (%w/w) | Comp2 (%w/w) |
|---|---|---|
| Glycerine | 1.5-6 | 1.5-6 |
| Pentylene glycol | 1.5-6 | 1.5-6 |
| C10-30 Alkyl Acrylate Cross-polymer | 0.5 | / |
| Xanthan gum | / | 1.0 |
| Benzoic acid | 0.1 -0.5 | 0.1 - 0.5 |
| Citric acid | 0.01 -0.1 | 0.01 -0.1 |
| Water | 87 - 96 | 87-95 |

### Example 2 - Viscosity analysis

All the compositions of Example 1 are in the form of a gel.

Their dynamic viscosity is measured on a Brookfield DV1 RV viscosimeter - Spindle 5 at 25°C at a speed of rotation of 50 rpm during 1 minute.

The results obtained are shown in Table 3.

**Table 3 - Viscosity of the compositions at 25°C**

| Composition | Viscosity (mPa.s) |
|---|---|
| C1 | 3472 |
| C2 | 5832 |
| C4 | 5544 |
| C5 | 3072 |
| Comp1 | 4144 |
| Comp2 | 1536 |

These results confirm that all the compositions according to the invention have a viscosity well above the target value of 2000 mPa.s. The viscosity of the comparative composition Comp2, using only xanthan gum, at the same concentration as *Caesalpinia spinosa* gum in compositions C1 and C2, is much lower than this target value.

All the tested compositions according to the invention are homogeneous (no sedimentation is visually observed following centrifugation at 4000 rpm for 20 minutes).

### Example 3 - Sensory profile

A monadic assessment of the sensory profiles of composition C2 according to the invention and comparative compositions Comp1 and Comp2 is carried out by 15 panelists trained to the sensory methodology for assessing topical products.

0.1 ml of the composition to be tested are applied onto each cheek.

Three assessment phases are evaluated:
- during application,
- 5 min upon application,
- 15 min upon application,

During the test, temperature and hygrometry of the room are checked, and lightening is controlled.

The results obtained are shown in figure 1. It is observed that the composition of the invention C2 is associated with a very good sensory profile, close to that of the composition Comp1 containing polyacrylates. No significant differences are found in the sensory characteristics of these two compositions

II is interesting to note that the comparative composition Comp2, containing xanthan gum, is far less satisfactory. It differs from the composition Comp1 by a wetter feel touch during application and 5 min upon application (criterion "Wetness"). Furthermore, a stronger tightening sensation is revealed 5 min upon application of the composition Comp2 (criterion "Tautness_5MIN"). Its absorption time is not as quick as that of the composition of the invention C2 (criterion "Absorption") and its stickiness is more important and more resistant in time (criteria "Stickiness").

### Example 4 - Moisturizing effects

This study is conducted on the composition according to the invention C3.

The aim of this study is to assess the power of skin moisturization of the composition through instrumental measurements of electrical capacitance (corneometry), after 15 min, 3, 6, 9 and 12 h, compared to the control area (without treatment), after one application of the composition according to the invention, on the forearm.

The efficacy through the subjective perception of the study subjects through self-assessments efficacy, after one application of the composition according to the invention, has been verified.

The subjects undergo a dermatological clinical assessment before application of the composition according to the invention (at T0) to verify the inclusion and non-inclusion criteria of the study. For the instrumental assessment of skin moisturization (corneometry), two areas of 25 cm² are demarcated on the anterior area of one of the subject's forearms. One site is used for the composition according to the invention application and the other one is kept as control (untreated area), randomly. For the self-assessment efficacy, the subjects apply the composition according to the invention only once on the anterior area of the other forearm, without a demarcated area, according to the sponsor's application mode and under technical supervision. Subjective perception assessments are performed through questionnaires immediately after application of the composition and 24 and 48 h after application. The subjects remain at rest in a room with controlled temperature and relative humidity for 30 min before the initial measurements and between the measurements.

The study is carried out in 22 women aged between 28 and 59 years.

### Electrical Capacitance Measurements

The measurements are performed by using the equipment Corneometer^{®} CM 825, through a measuring probe.

Initial measurements are performed to confirm the inclusion criteria of dry skin on the forearm. These measurements are considered as the T0 in the data analysis, for the approved subjects.

The readings are taken by placing the probe to the test sites with the pressure allowed by the spring (3.5 N). Four measurements are performed on each area. The readings indicate the degree of moisture on the skin surface based on variations in electric capacity. The apparatus scale is arbitrary, i.e., greater reading values indicate greater moisturization.

The subjects are kept in a room with controlled temperature and relative humidity (20°C ± 2°C and 50% ± 5 RH) for a period of 30 min before the measurements are taken and between the measurements. During this period, they remain seated in chairs arranged in the room.

On the anterior area of one of the forearms, two areas are demarcated.

One area is used for the application of the investigational composition and one area is kept as a control (no treatment). The allocation is randomized.

The measurements are performed at the following time-points:
- T0 - initial, before the investigational composition application;
- T15min - 15 minutes (+ 5 min) after investigational composition application.
- T3h - 3 hours (± 10 min) after investigational composition application.
- T6h - 6 hours (± 10 min) after investigational composition application.
- T9h - 9 hours (± 30 min) after investigational composition application.
- T12h - 12 hours (± 30 min) after investigational composition application. Statistical analysis is performed by Student t test.

The results of the electrical capacitance measurements, expressed in arbitrary unit, are shown in Table 4.

**Table 4 - Electrical capacitance of the skin before (T0) or after application of the composition C3 according to the invention**

| Time | T0 | T15min | T3h | T6h | T9h | T12h |
|---|---|---|---|---|---|---|
| Mean | 24.5 | 31.9 | 26.2 | 26.3 | 24.3 | 24.3 |
| Standard error | 1.6 | 1.9 | 1.4 | 1.3 | 1.2 | 1.2 |
| p-value | - | <0.001 | 0.029 | 0.014 | 0.603 | 0.592 |

These results demonstrate that the composition of the invention C3 has promoted a significant increase in moisturization of the skin, immediately after its application and up to at least 6 hours after the application.

In comparison, no significant increase in moisturization was observed immediately and after 3, 6, 9, 12, 24 and 48 hours for the control area.

### Self-assessment efficacy

Self-assessment efficacy performed by the study subjects, immediately after application, gives the following results, expressed as agreement percentages:
- the composition evens the skin: 90.9%,
- the composition has a refreshing texture: 86.4%,
- the composition has a non-oily texture: 90.9%,
- the composition gives a non-sticky finish: 95.5%,
- the composition has an invisible finish: 90.9%,
- the composition has immediate absorption: 90.9%,
- the composition gives a clean skin sensation: 95.5%,
- the composition gives a finish without leaving residues: 90.9%,
- the texture of the composition is ultra-light: 95.5%,
- the composition has a watery texture (aqua-like): 68.2%.

### Example 5 - Rheological assessment

The composition of the invention C5 and the comparative composition based on acrylate cross-polymer Comp1 are subjected to an oscillatory stress amplitude sweep test.

In the oscillatory amplitude stress sweep test at low amplitudes, viscoelasticity is evaluated, i.e., the relative importance of elastic and viscous effects of a given fluid. This test allows correlating the microstructure with the mechanical response.

The solid-like component at any particular frequency is characterized by the storage modulus, G', and the liquid-like response is described by the complementary loss modulus, G". The units of both these moduli are Pascals (Pa), and their values vary with the applied frequency, ω, which is given by 2πf, where f is the frequency in hertz (Hz).

Regarding cosmetic compositions:
- if a composition only has a viscous behavior, it means that it behaves as a pure liquid and it flows without any resistance. This is not desired since the consumer will face difficulties in handling the product and spreading it in the skin;
- on the other hand, if it only has an elastic component, it behaves as a rigid solid, which is also not interesting to consumer experience.

This test is carried out on a stress-controlled rheometer such as AR-G2 or DHR-3 from TA Instruments, at 25°C. It consists in imposing a constant frequency (f =1 Hz) and evaluating how the storage modulus G' and the loss modulus G" behave as the stress amplitude is varied.

The results are shown in figure 2, in a/ for the composition of the invention C5 and in b/ for the comparative composition Comp1.

It is observed for both compositions three delimited regions, namely the linear viscoelastic regime (LVR), the yielding region, and the non-linear region. The LVR occurs in the low stress amplitude range and is characterized by constant moduli. Both the yielding and the non-linear region are in the domain of the so called "large amplitude oscillatory shear".

These observations denote a viscoelastic behavior of the compositions.

## Claims

1. Cosmetic composition in the form of an aqueous gel, **characterized in that** it comprises, in percentage by weight relative to the total weight of the composition, at least 80 % water and 0.5 to 2 % *Caesalpinia spinosa* gum, **in that** it has a dynamic viscosity at 25°C higher than or equal to 2000 mPa.s, and **in that** it is devoid of acrylate.

2. Composition according to claim 1, which is devoid of fatty ingredients or comprises less than 2 % by weight of fatty ingredients relative to the total weight of the composition.

3. Composition according to any of claims 1 or 2, comprising xanthan gum.

4. Composition according to claim 3, comprising 0.01 to 1 % by weight, relative to the total weight of the composition, of xanthan gum.

5. Composition according to claim 3 or 4, comprising 0.55 to 1 % by weight, relative to the total weight of the composition, of the mixture of *Caesalpinia spinosa* gum and xanthan gum.

6. Composition according to any of claims 3 to 5, wherein the weight ratio *Caesalpinia spinosa* gum / xanthan gum is between 75/25 and 90/10.

7. Composition according to any of claims 1 to 6, comprising an additional cosmetically active ingredient.

8. Composition according to any of claims 1 to 7, comprising at least one humectant, preferably at least two humectants.

9. Composition according to claim 8, comprising as humectant(s) at least glycerin, preferably in combination with pentylene glycol.

10. Composition according to any of claims 7 to 9, comprising hyaluronic acid or a cosmetically acceptable salt thereof.

11. Composition according to any of claims 1 to 10, which is devoid of cationic surfactants.

12. Composition according to any of claims 1 to 11, which is a leave-on composition.

13. Use of a composition according to any of claims 1 to 12 for the cosmetic non-therapeutic treatment of the skin of a subject.

14. Cosmetic non-therapeutic method for treating the skin of a subject, comprising topically applying the composition according to any of claims 1 to 12 to a surface of said skin.

15. Method according to claim 14, which is devoid of rinsing step after said topically applying said composition to a surface of said skin.
